# EUROPEAN PATENT APPLICATION

(11) **EP 4 193 910 A1**
(43) Date of publication of application: **14.06.2023**
(21) Application number: 21213940.6
(22) Date of filing: 13.12.2021
(51) Int. Cl.: A61B 5/00

(54) **METHOD AND SYSTEM FOR ASSESSMENT AND PREDICTING SLEEPINESS DURING OSA SCREENING USING CONSUMER DEVICES**

(71) Applicant: Koninklijke Philips N.V., 5656 AG Eindhoven (NL)
(72) Inventor: WEIJSEN, Tim Elisabeth Joseph, Eindhoven (NL); VAN EE, Raymond, Eindhoven (NL); LEMMENS, Paul Marcel Carl, Eindhoven (NL); MENA BENITO, Maria Estrella, Eindhoven (NL); WESTERINK, Joanne Henriëtte Desirée Monique, Eindhoven (NL); LEUFKENS, Timmy Robertus Maria, Eindhoven (NL); TEN KATE, Warner Rudolph Theophile, Eindhoven (NL)
(74) Representative: Philips Intellectual Property & Standards

(57) **Abstract**

The present invention relates to sleepiness assessment. In order to provide a more accurate measure to predict OSA severity, an apparatus is provided for sleepiness assessment. The apparatus comprises an input unit, a processing unit, and an output unit. The input unit is configured to receive data indicative of an activity currently performed by a user. The processing unit is configured to determine, based on the activity currently performed by the user, a current situation the user is engaging in. The processing unit is configured to determine whether the current situation matches one of a plurality of pre-determined situations used for situational sleepiness assessment. In response to the determination that the current situation matches one of the plurality of predetermined situations, the processing unit is configured to send a notification to the user for a self-report of a user's current subjective sleepiness level and/or obtain a user's current objective sleepiness level from sensor data. The processing unit is further configured to generate, based on the determined situation and the user's current subjective and/or objective sleepiness level, a situational sleepiness profile indicative of a sleep disturbance on daytime sleepiness in specific situations. The output unit is configured to provide the generated situational sleepiness profile, which is preferably useable to support sleep apnea diagnostics.

## Description

### FIELD OF THE INVENTION

The present invention relates to sleepiness assessment, and in particular to an apparatus for sleepiness assessment, to a system for generating a treatment plan, to a method for sleepiness assessment, to a method for generating a treatment plan, to a computer program product, and to a computer-readable data carrier.

### BACKGROUND OF THE INVENTION

Obstructive Sleep Apnea (OSA) is a chronic condition characterized by frequent episodes of upper airway collapses during sleep. Increasingly, OSA is being recognized as an independent risk factor for several clinical consequences, including systemic hypertension, cardiovascular disease, stroke, and abnormal glucose metabolism.

The effects of OSA on nocturnal sleep quality and ensuing daytime fatigue and sleepiness are widely acknowledged and Excessive daytime sleepiness is regarded as the most common and most important symptom of OSA, although the predictive value for OSA severity (Apnea Hypopnea Index, AHI) is rather low. Sleepiness is defined as the inability to maintain wakefulness and alertness during the major waking episodes of the day, with sleep occurring unintentionally or at inappropriate times.

Numerous randomized controlled trials have demonstrated a significant improvement in daytime sleepiness when such patients are effectively treated with Continuous Positive Airway Pressure (CPAP) as compared to sham CPAP or oral placebo. Daytime sleepiness is related to OSA and snoring in general population studies. In the Wisconsin Sleep Cohort Study, about 23% of the women with an AHI of ≥5 reported excessive daytime sleepiness compared with only 10% of non-snoring women. The corresponding prevalence in men was 16% and 3% respectively. Similar findings were reported from the Sleep Heart Health Study using the ESS with a significant, progressive increase in sleepiness with increasing AHI in both older and younger subjects and independent of gender, age, and BMI.

Perhaps sounding slightly contradictory, the aforementioned evidence of sleep apnea induced daytime sleepiness is weak, as only a fraction of patients with OSA in the population report daytime sleepiness. Attempts to find the suggested association between the arousals and sleepiness have also failed. Daytime sleepiness can be due to a number of factors and OSA patients may have suffered from other disorders causing sleepiness. The association between OSA and sleepiness is also less evident in patients with chronic diseases such as in patients with congestive heart failure who report less daytime hypersomnolence regardless of whether they have OSA or not.

Currently, assessment of excessive sleepiness can be achieved by means of methods such as the Epworth Sleepiness Scale or the Karolinska Sleepiness Scale (KSS). The Epworth Sleepiness Scale is a scale widely used in the field of sleep medicine as a subjective measure of a patient's sleepiness. The test is a list of eight situations in which you rate your tendency to become sleepy on a scale of 0, no chance of dozing, to 3, high chance of dozing in situations like sitting and reading, watching TV and other activities. Another method is the Karolinska Sleepiness Scale. This scale measures the subjective level of sleepiness at a particular time during the day. Subjects indicate which level best reflects the psycho-physical sate experienced in the last 10 min.

However, both methods may be limited in providing a sufficiently accurate measure to predict OSA severity.

### SUMMARY OF THE INVENTION

There may, therefore, be a need to provide a more accurate measure to predict OSA severity.

The object of the present invention is solved by the subject-matter of the appended independent claims, wherein further embodiments are incorporated in the dependent claims.

According to a first aspect of the present invention, there is provided an apparatus for sleepiness assessment. The apparatus comprises an input unit, a processing unit, and an output unit. The input unit is configured to receive data indicative of an activity currently performed by a user. The processing unit is configured to determine, based on the activity currently performed by the user, a current situation the user is engaging in. The processing unit is configured to determine whether the current situation matches one of a plurality of pre-determined situations used for situational sleepiness assessment. In response to the determination that the current situation matches one of the plurality of predetermined situations, the processing unit is configured to send a notification to the user for a self-report of a user's current subjective sleepiness level and/or obtain a user's current objective sleepiness level from sensor data. The processing unit is further configured to generate, based on the determined situation and the user's current subjective and/or objective sleepiness level, a situational sleepiness profile indicative of a sleep disturbance on daytime sleepiness in specific situations. The output unit is configured to provide the generated situational sleepiness profile, which is preferably useable to support sleep apnea diagnostics.

The apparatus as described herein provides one or more assessments of situational sleepiness during specific situations, possibly using self-learning. For example, the apparatus as described herein may assess sleepiness during specific daily situations like watching TV and driving in a car (e.g.), based on input from connected measures (e.g. GPS location, type of activity, body position). A specific desired situation for data collection can be recognized by the apparatus and a push notification may be triggered by the apparatus via a connected smartphone app to assess the user's current perception of sleepiness. The user's current objective sleepiness level may be collected to enrich or replace the user's perception of sleepiness. For example, when the user does not respond to the request for information, the subjective input may be replaced by the objective measure for that specific moment in time. The situational sleepiness profile may be generated based on the collected information, which can be used as a metric that supports the diagnosis of sleep apnea.

Assessment of sleepiness in this manner may enable a better prediction of the moment and level of subjective sleepiness level, which may be used to support sleep apnea diagnostics given its higher clinical relevance in predicting OSA severity. An additional benefit of having multiple measures for identical situations is that the sleepiness level can be correlated to nocturnal sleep measures, yielding options for improving diagnostics.

According to an example of the present disclosure, if it is determined that the current situation has a duration exceeding a threshold, the processing unit is configured to send a plurality of notifications to the user for a self-report of the user's subjective sleepiness levels throughout the duration of the current situation or obtain a plurality of user's objective sleepiness levels from the sensor data throughout the duration of the current situation.

Specific situations may need to have a minimum duration of a predefined time before the processing unit instructs the app to send out a push notification. When a specific situation is known to last for longer periods of time, it may be relevant to prompt for multiple indications of sleepiness throughout the duration of the situation.

According to an example of the present disclosure, the processing unit is configured to send the notification to the user for a self-report of a score indicative of the user's subjective sleepiness level.

Combining the features of the two measures (limited number of situational questions and a scoring method) may result in a benefit for the patient, since limited questions have to be answered to provide insight into sleepiness by only posing the question for any / a specific situation that is relevant at that specific point in time. This may enable more a fine-grained assessment of sleepiness during the various situations.

According to an example of the present disclosure, the processing unit is configured to obtain the user's objective sleepiness level from sensor data, if no self-report of the user's subjective sleepiness level is received.

In other words, when the user does not respond to the request for information, the subjective input may be replaced by the objective measure for that specific moment in time.

According to an example of the present disclosure, the processing unit is configured to determine, based on the received data, a moment that the user is less engaged in the current situation. The processing unit is configured to send the notification to the user at the determined moment.

When a specific situation is known to last for longer periods of time, the moments for prompting may be based on common patterns in the data. For instance, for watching TV, a proper moment to prompt for a score could be during a commercial break that can be detected from an increase in volume in the room. In this way, there may be less delay between the prompt and receiving the user's subjective sleepiness level.

According to an example of the present disclosure, the processing unit is configured to monitor a delay between sending the notification and receiving the user's self-reported sleepiness level and determine whether the delay exceeds a threshold. In response to the determination that the delay exceeds the threshold, the processing unit is configured to adjust, based on the delay, a moment for sending the notification to the user.

For instance, by monitoring the delay between the prompt and receiving the score, and storing that a specific pattern in the sensors' data is unsuitable for prompting the user for a score and delaying the prompt until a pattern more suited is recognized.

According to an example of the present disclosure, the input unit is configured to receive data indicative of mental functioning of the user. The provided situational sleepiness profile further comprises the information on mental functioning of the user.

In other words, information on mental function (e.g. stress level) may be measured with a connected wearable. This could be used as an indication of the 'burden' level that the patient is experiencing, replacing or enriching input from questions like 'How much is it a burden for you?', related to the suffering from the experienced sleep disorder.

According to an example of the present disclosure, the input unit is configured to receive data indicative of activities the user performs from day to day over a predefined period. The processing unit is configured to determine, based on the received data, a daily routine motion pattern of the user over the predefined period, wherein the daily routine motion pattern is indicative of an activity level over a day. The processing unit is configured to determine, based on the daily routine motion pattern of the user, a low motion moment that happens repeatedly for a plurality of days over the predefined period. At the low motion moment, the activity level is lower than a threshold. The processing unit is further configured to send a notification to prompt the user for indicating whether the user did a nap at the low motion moment. In response to a user's response indicating that the user did a nap at the low motion moment, the processing unit is configured to provide information about the low motion moment to the situational sleepiness profile.

For example, an artificial learning system may learn from objectively measured behavioral routine activity patterns of the user detected by a motion sensor and/or sedentary behavior detector. E.g. one could detect lack of motion for a period of half an hour at about 3pm in the afternoon, particularly when this happens repeatedly for several days. The low motion moment may have an activity level lower than a threshold. The low motion moment is typically shown as relative dips in routine motion patterns. The user will afterwards be asked whether s/he did a nap at this particular moment. If yes, this information can be used to predict future nap periods. It is possible to base the assessment upon a predetermined number or periods for calibration.

According to an example of the present disclosure, the input unit is configured to receive data comprising previous measurement of activities of the user and an associated situational sleepiness profile. The processing unit is configured to predict a moment when sleepiness is likely to have changed from the previous measurement and send a notification to the user for a self-report of the user's sleepiness level at the determined moment.

In other words, it is also possible to predict which moments will bring relevant sleepiness information based on the responses and behavior patterns recorded so far. The system could predict when sleepiness is likely to have changed from the previous measurement and send a notification for a self-report based on that. Both moments of expected high sleepiness and expected low sleepiness could be included. This will enhance the detail gathered about the sleepiness patterns of the user.

According to an example of the present disclosure, the data indicative of an activity currently performed by the user comprises sensor data obtained from one or more sensors for monitoring an activity of the user, sensor data obtained from one or more sensors for monitoring an environmental parameter in a user's location, data obtained from one or more smart-home devices, data indicative of location information of the user, or any combination thereof.

According to a second aspect of the present invention, there is provided a system for generating a treatment plan. The system comprises a sensor configured to measure an apnea hypopnea index (AHI) of a user, an apparatus according to the first aspect and any associated example configured to measure a sleep disturbance on daytime sleepiness in specific situations, and a treatment plan generation device configured to establish a treatment plan by linking nocturnal sleep behavior related to AHI to the impact of the sleep disturbance on daytime sleepiness in specific situations.

In other words, the collected information may be used to establish specific treatment plans, by linking nocturnal sleep behavior related to OSA (AHI), to the impact of the sleep disturbance on daytime sleepiness in specific situations, perhaps yielding alternative solutions or settings in the treatment plan (triaging engine). The specific manner to better triage could come over time, when sufficient data on sleepiness measures and treatment success can be evaluated.

According to a third aspect of the present invention, there is provided a method for sleepiness assessment. The method comprises:
- receiving data indicative of an activity currently performed by a user;
- determining, based on the activity currently performed by the user, a current situation the user is engaging in;
- determining whether the current situation matches one of a plurality of pre-determined situations used for situational sleepiness assessment;
- in response to the determination that the current situation matches one of the plurality of predetermined situations, sending a notification to the user for a self-report of the user's current sleepiness level and/or obtaining the user's current drowsiness level from sensor data; and
- generating, based on the determined situation and the user's current subjective and/or objective sleepiness level, a situational sleepiness profile indicative of a sleep disturbance on daytime sleepiness in specific situations; and
- providing the generated situational sleepiness profile, which is preferably useable for sleep apnea diagnostics.

The method for sleepiness assessment may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

According to a fourth aspect of the present invention, there is provided a method for generating a treatment plan. The method comprises:
- measuring an apnea hypopnea index (AHI) of a user;
- measuring a sleep disturbance on daytime sleepiness in specific situations according to the method of the third aspect and any associated example; and
- establishing a treatment plan by linking nocturnal sleep behavior related to AHI to the impact of the sleep disturbance on daytime sleepiness in specific situations.

The method for generating a treatment plan may also be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

According to another aspect of the present invention, there is provided a computer program product comprising instructions to cause the apparatus according to the first aspect and any associated example to execute the steps of the method of the third aspect and any associated example, or comprising instructions to cause the system of the second aspect and any associated example to execute the steps of the method of the fourth aspect and any associated example.

According to a further aspect of the present invention, there is provided a computer-readable data carrier having stored there on the computer program product.

As used herein, the term "activity performed by a user" may include activities that are actively performed by the user, such as running, watching TV, driving a car, etc. The term "activity performed by a user" may also include other activities that the user is engaged in, such as taking a bus or taxi as a passenger in a transportation activity.

### BRIEF DESCRIPTION OF THE DRAWINGS

These and other aspects of the invention will be apparent from and elucidated further with reference to the embodiments described by way of examples in the following description and with reference to the accompanying drawings, in which
Fig 1 illustrates an exemplary apparatus for sleepiness assessment.
Fig. 2 illustrates a pattern of activity levels as monitored over the day of a user.
Fig. 3A-3C illustrate a two-process model of sleep regulation.
Fig. 4 illustrates an exemplary system for sleepiness assessment, which may also be used for generating a treatment plan.
Fig. 5 illustrates a flow chart describing an exemplary method for sleepiness assessment.
Fig. 6 illustrates a flow chart describing an exemplary method for generating a treatment plan.

### DETAILED DESCRIPTION OF EMBODIMENTS

As mentioned, the current methods may be limited in providing a sufficiently accurate measure to predict OSA severity. For example, the Epworth Sleepiness Scale Method may have the shortcoming of using retrospective/reflective self-reporting. Self-reporting requires a substantial level of awareness and ability to reflect back to specific situations assessed by the aforementioned scale. The Karolinska Sleepiness Scale (KSS) overcomes the shortcoming of the ESS since patients reflect their current state. However, this method may suffer the disadvantage of being sensitive to fluctuations because it measures situational sleepiness, which is not taken into account by this specific measure.

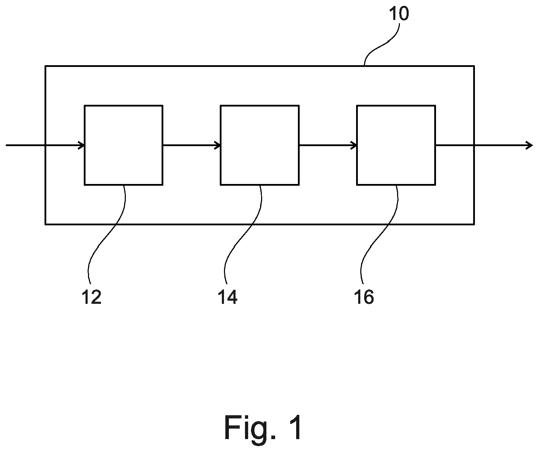

Towards this end, an apparatus is proposed for sleepiness assessment. An exemplary apparatus 10 is shown in Fig. 1. The exemplary apparatus 10 comprises an input unit 12, one or more processing units 14, and an output unit 16.

In general, the apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 1 may show a limited number of components by way of example, it can be appreciated that a greater or a fewer number of components may be employed for a given implementation.

In some implementations, the apparatus 10 may be embodied as, or in, a device or apparatus, such as a server, workstation, or mobile device. The apparatus 10 may comprise one or more microprocessors or computer processors, which execute appropriate software. The processing unit 14 of the apparatus 10 may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the apparatus 10 may be implemented with or without employing a processor, and also may be implemented as a combination of dedicated hardware to perform some functions and a processor (e.g., one or more programmed microprocessors and associated circuitry) to perform other functions. For example, the functional units of the apparatus 10, e.g., the input unit 12, the one or more processing units 14, and the output unit 16 may be implemented in the device or apparatus in the form of programmable logic, e.g., as a Field-Programmable Gate Array (FPGA). In general, each functional unit of the apparatus may be implemented in the form of a circuit.

In some implementations, the apparatus 10 may also be implemented in a distributed manner. For example, some or all units of the apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

The input unit is configured to receive data indicative of an activity currently performed by a user via a wired and/or wireless connection.

In some examples, the received data may comprise sensor data acquired by one or more sensors. The one or more sensors may comprise on-body sensors and/or on-object sensors.

### On-bodv sensors

The on-body sensor may refer to a sensor that is attachable or connectable to the user. The on-body sensors may also be referred to as wearable sensors. Examples of the on-body sensors may include, but are not limited to, inertial sensors (e.g. accelerometer, gyroscopes, pressure sensors, magnetic field sensors, or other inertial sensors), location sensors (e.g. GPS), physiological sensors (e.g. blood pressure cuff, electrocardiogram, spirometer, electrooculography, skin temperature sensor).

The inertial sensors may be attached over an individual's body and used to measure the acceleration and angular velocity of an object along three mutually perpendicular axes. For example, an accelerometer may be used for monitoring dynamic activities, e.g. for distinguishing static postures (e.g. laying, standing, or sitting). Gyroscopes may be used as an additional method for measuring rotational movements, for detecting behaviors like falling by measuring the user's angular velocity of movement such as bending knees. Magnetic field sensors may be placed close to the measurement location and thus to detect a user's direction. For example, when recognizing "watching TV", a magnetometer may tell that the user is facing the direction of the television whilst combing accelerometers and indoor localization information.

Location sensors (e.g. GPS) may be used to track user movement activities including location, distance, and speed. With the location sensors, it is possible to determine transportation of the user, such as driving, cycling, or taking a bus.

Physiological sensors may be also used for monitoring the user's activity. For example, when the user starts performing intensive activities such as running and swimming, their heart rate will increase.

### On-obiect sensors

On-object sensors, on the other hand, may refer to a sensor that is attachable to an object, such as sensors attached to a wall in a room, sensors in a smartphone, sensors in a smart speaker, sensors in a smart home device, etc. On-object sensors may be used to monitor composite activity like watching TV, or preparing a meal. Examples of the on-object sensors may include, but are not limited to, environmental sensors (e.g. thermometer, hygrometer, and/or energy sensor), location detectors (e.g. infrared), binary sensors and tags (active RFID, RFID tags, NFC tags).

Environmental sensors may be used to measure indoor environmental conditions such as humidity, temperature, energy, and sound. For example, a sound sensor may be used to detect whether the user is talking or watching TV.

Location detectors, such as Passive InfraRed (PIR) sensor, may be used to monitor human indoor localization and therefore a pattern of activity levels over the day of the user.

Binary sensors may be used to sense an object's state with a digit of 0 or 1, representing on/off or open/close, such as for detecting window open/close state, door open/close state, light on/off state, remote control on/off state, etc.

RFID tags and readers may be used to detect human object interactions in the matter of motion and touch.

In some examples, the received data may comprise environmental information obtained via a SmartHome interface, such as TV on/off status, lights on/off settings, etc.

The received data may be used for detecting various activities including, but not limited to, aerobic exercises (e.g. walking, jogging, climbing, descending, running, or swimming), transportation (driving, cycling, or taking a bus), sedentary postures (e.g. sitting, lying, standing, or tilting), transitional activities (e.g. sit-to-stand, stand-to-walk, walk-to-run, or run-to-walk), daily lives (e.g. watching TV or cooking), and ball sports (e.g. playing football).

The processing unit 14 is configured to determine, based on the activity currently performed by the user, a current situation the user is engaging in, and whether the current situation matches one of a plurality of pre-determined situations used for situational sleepiness assessment. Examples of the pre-determined situations used for situational sleepiness assessment may include, but are not limited to, sitting and reading, watching TV, sitting inactive in a public place (e.g. a theatre or a meeting), as a passenger in a car for an hour without a break, lying down to rest in the afternoon when circumstances permit, sitting and talking to someone, sitting quietly after a lunch without alcohol, and in a car while stopped for a few minutes in traffic.

In response to the determination that the current situation matches one of the plurality of predetermined situations, the processing unit 14 is configured to send a notification to the user for a self-report of a user's current subjective sleepiness level and/or obtain a user's current objective sleepiness level from sensor data.

For example, the processing unit 14 may collect information about the current state and environments (e.g. at home, work) of the user from the received data. The processing unit 14 may then establish a current situation of the user and determine whether e.g. the user is watching TV, driving a car, or is engaged in other activities, actively moving, or being sedentary. When a specific situation is confirmed by the processing unit 14, a push notification may be sent to the user, e.g. via a smartphone app, with the request to report the subjective sleepiness level at that specific moment.

In some examples, the processing unit 14 may be configured to send the notification to the user for a self-report of a score indicative of the user's sleepiness level. For example, the scoring method in the KSS may be used, which spans 9 levels (1 = extremely alert, 2 = very alert, 3 = alert, 4 = rather alert, 5 = neither alert nor sleepy, 6 = some signs of sleepiness, 7 = sleepy, but no effort to keep awake, 8 = sleepy, some effort to keep awake, 9 = very sleepy, great effort keeping awake, fighting sleep). The smartphone app may present the list of scores such that the user can select one from the list representing the user's current subjective sleepiness level. Combining the features of the two measures (situational questions and scoring method) may result in a benefit for the patient, since limited questions have to be answered to provide insight into sleepiness by only posing the question for any / a specific situation listed in the pre-determined situations that is relevant at that specific point in time. Summarizing, the combination of these measures enables improved accuracy in OSA screening because the KSS has a more granular scoring than the ESS has. This enables more fine-grained assessment of sleepiness during the various situations listed in the ESS.

Specific situations may need to have a minimum duration of a predefined time before the processing unit instructs the app to send out a push notification. If it is determined that the current situation has a duration exceeding a threshold, the processing unit 14 may be configured to send a plurality of notifications to the user for a self-report of the user's subjective sleepiness levels throughout the duration of the current situation and/or obtain the user's objective sleepiness levels throughout the duration of the current situation. For example, when a specific situation is known to last for longer periods of time, it may be relevant to prompt for multiple scores of sleepiness throughout the duration of the situation. A maximum number of notifications may be predetermined, so as not to send messages too often. Not only should the activity last longer than the threshold, also after this threshold has been reached, the messages should not be sent too often.

When a specific situation is known to last for longer periods of time, the moments for prompting may be determined based on common patterns in the data. For example, the processing unit 14 may be configured to determine, based on the received data, a moment that the user is less engaged in the current situation. The processing unit may be configured to send the notification to the user at the determined moment. For example, the moments for prompting may be based on common patterns in the data. For instance, for TV viewing, a proper moment to send a notification to the user could be during a commercial break that can be detected from an increase in volume in the room, as this may indicate that the user is less engaged in watching TV. In this way, the delay between the prompt and receiving the user's self-reported subjective sleepiness level may be shortened.

Over time, the prompting can be personalized e.g. based upon a personalized self-learning system. For example, the processing unit 14 may be configured to monitor a delay between sending the notification and receiving the user's self-reported subjective sleepiness level and determine whether the delay exceeds a threshold. In response to the determination that the delay exceeds the threshold, the processing unit 14 may be configured to adjust, based on the delay, a moment for sending the notification to the user. For example, the processing unit 14 may monitor the delay between the prompt and receiving the score, store that a specific pattern in the sensors' data is unsuitable for prompting the user for a score, and delay the prompt until a pattern more suited is recognized.

The objective sleepiness level may be derived from sensor data acquired e.g. by a wearable (e.g. wrist worn) device with e.g. photoplethysmogram (PPG). The objective drowsiness level from the sensor data may replace or enrich the user's self-reported subjective sleepiness level. For example, this information may further support the right moment for triggering notifications to the user to subjectively assess sleepiness level, or when the user does not respond to the request for information, replace the subjective input by the objective measure for that specific moment in time.

The processing unit 14 is further configured to generate, based on the determined situation and the user's current subjective and/or objective sleepiness level, a situational sleepiness profile indicative of a sleep disturbance on daytime sleepiness in specific situations. As discussed hereinbefore, on-body sensor(s), on-objection sensor(s) and/or monitoring unit(s) may be used to track the user's activity continuously. Therefore, various situations and associated subjective and/or objective sleepiness levels may be continuously collected and stored in the user's situational sleepiness profile. Therefore, the user's situational sleepiness profile may comprise previously and currently recorded situations and associated subjective and/or objective sleepiness levels.

The output unit 16 is configured to provide the generated situational sleepiness profile e.g. to a display or to a database. The generated situational sleepiness profile is preferably useable to support sleep apnea diagnostics.

Besides sending a notification to prompt the user for indicating current subjective sleepiness level, during a predefined period (e.g. the first week of the assessment), some additional questions may be sent to the user to confirm the estimated current state and environments. The information may be used to improve by means of AI self-learning the accuracy of measurements over the course of assessment (typically within a 2-4 weeks' timeframe). Then, the system and method as described herein may store all the collected data in a database. The information may be used to classify sleepiness at the specific situations/states (which could be named 'events').

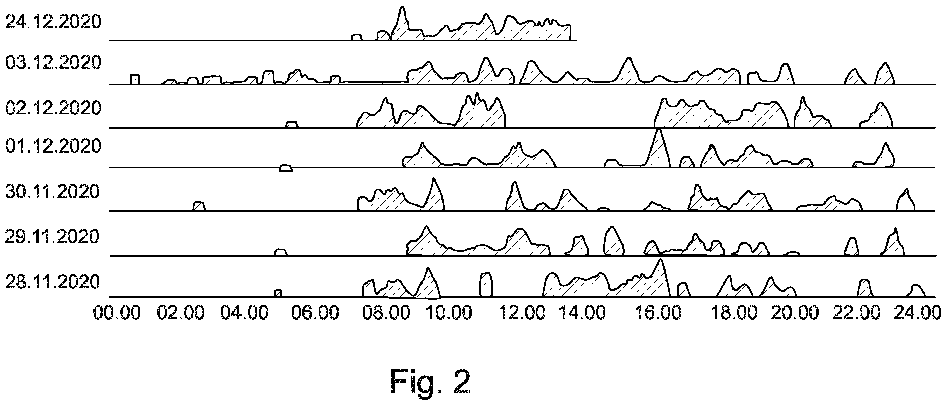

The moments for sending the additional questions may be determined in the following way. The input unit 12 may receive data indicative of activities the user performs from day to day over a predefined period. The data may be acquired by a sensor, such as one or more above-described on-body sensors and/or on-board sensors. The processing unit 14 may determine, based on the received data, a daily routine motion pattern of the user over the predefined period. The daily routine motion pattern is indicative of an activity level over a day. As an example, Fig. 2 shows a pattern of activity levels as monitored over the day of a user. The graphs are obtained using Passive InfraRed (PIR) sensors. These are mounted at the wall, and it is assumed every room is equipped with one, at least in those the user is commonly visiting during the day. Alternatively, the user could wear a smartwatch or alike device, where the accelerations sensed by the corresponding sensor in the watch may be measured to arrive at a similar graph. The processing unit may determine, based on the daily routine motion pattern of the user, a low motion moment that happens repeatedly for a plurality of days over the predefined period. At the low motion moment, the activity level is lower than a threshold. The low motion moment may also be referred to as a low motion moment, which may be shown as relative dips in routine motion patterns. From the graph in Fig. 2, as an example, it can be inferred to ask for a sleepiness update around 14:00 and during 20:00-22:00. The 14:00 inquiry would be skipped on 28th and 29th, while on 3rd the inquiry could be triggered at about 15:00, when activity levels up again ('Did you take a nap?'). The processing unit 14 may send a notification to prompt the user for indicating whether the user did a nap at the low motion moment. In response to a user's response indicating that the user did a nap at the low motion moment, the processing unit 14 may provide information about the low motion moment to the situational sleepiness profile. In other words, the user will afterwards be asked whether s/he did a nap at this particular moment. If yes, this information can be used to predict future nap periods. We can base the assessment upon a predetermined number or periods for calibration.

In addition to the information about the user's current subjective and/or objective sleepiness level, information on mental function (e.g. stress level) may also be measured with a connected wearable. This could be used as an indication of the 'burden' level that the patient is experiencing, replacing, or enriching input from questions like 'How much is it a burden for you?', related to the suffering from the experienced sleep disorder.

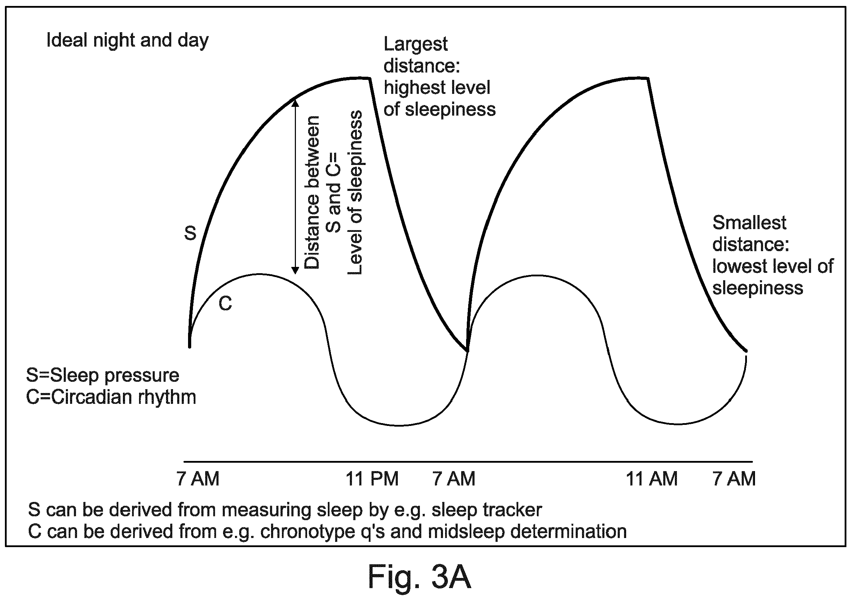

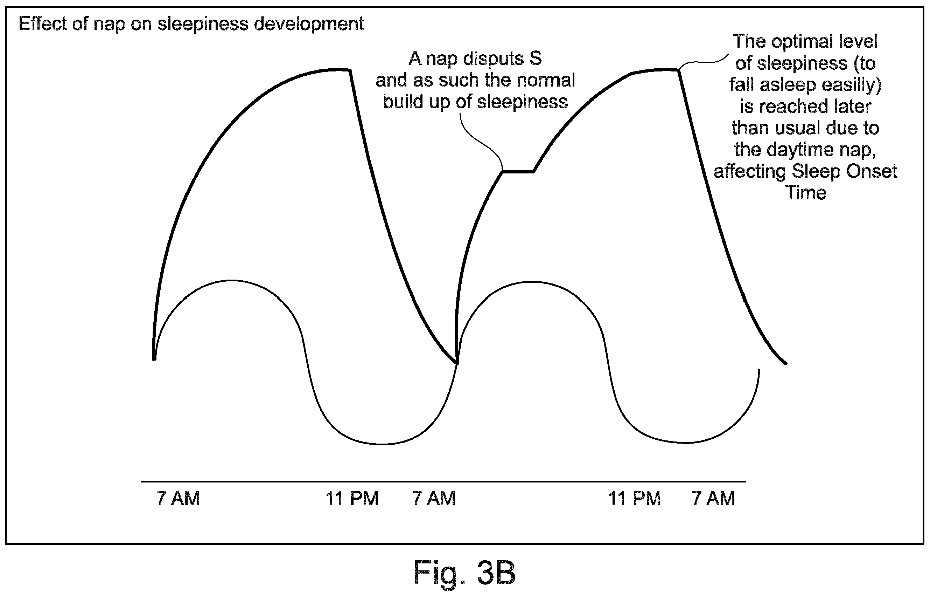

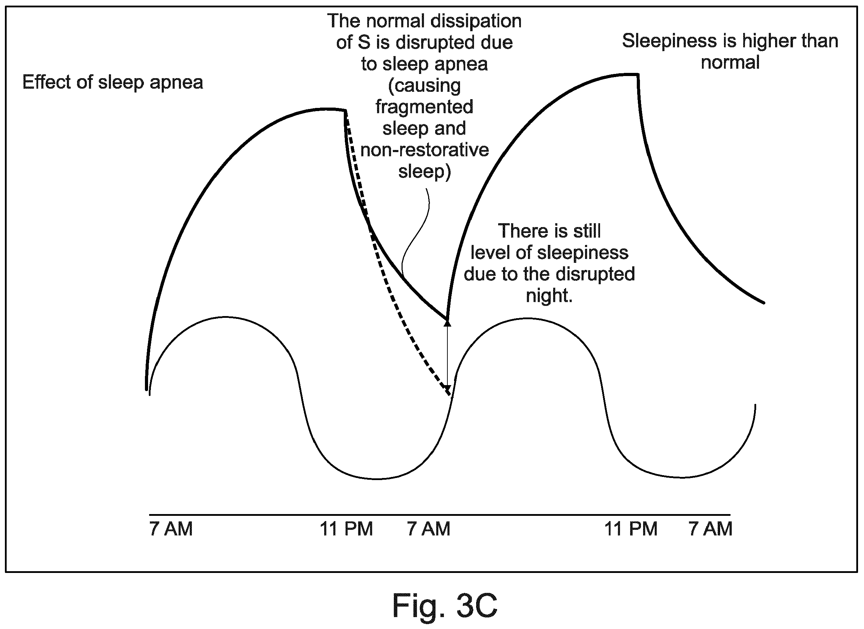

It is also possible to predict which moments will bring relevant sleepiness information based on the responses and behavior patterns recorded so far. The system could predict when sleepiness is likely to have changed from the previous measurement and send a notification for a self-report based on that. Both moments of expected high sleepiness and expected low sleepiness could be included. This will enhance the detail gathered about the sleepiness patterns of the user. A method to predict the sleepiness level from a user can be based on the conventional two-process model of sleep regulation, where the circadian sleep rhythm of a user (based on measured sleep behavior) can be used as input. Additionally, naps typically seen in sleep apnea patients influence these patterns and can be taken into account when making a prediction of the sleepiness level. Fig. 3A to 3C shows a two-process model of sleep regulation, illustrating the influence on sleepiness level of a user in the situation of a healthy circadian sleep rhythm (see Fig. 3A), healthy circadian sleep rhythm with a nap (see Fig. 3B), and the effect of sleep apnea on sleepiness (see Fig. 3C). The moments where the model predicts the highest level of sleepiness (see Fig. 3, largest distance between S and C), can be initially used as a trigger to send the notification to the user for a self-report of the sleepiness level. Over time, the system can make slight adjustments to the model and improve the output for a more accurate prediction of sleepiness.

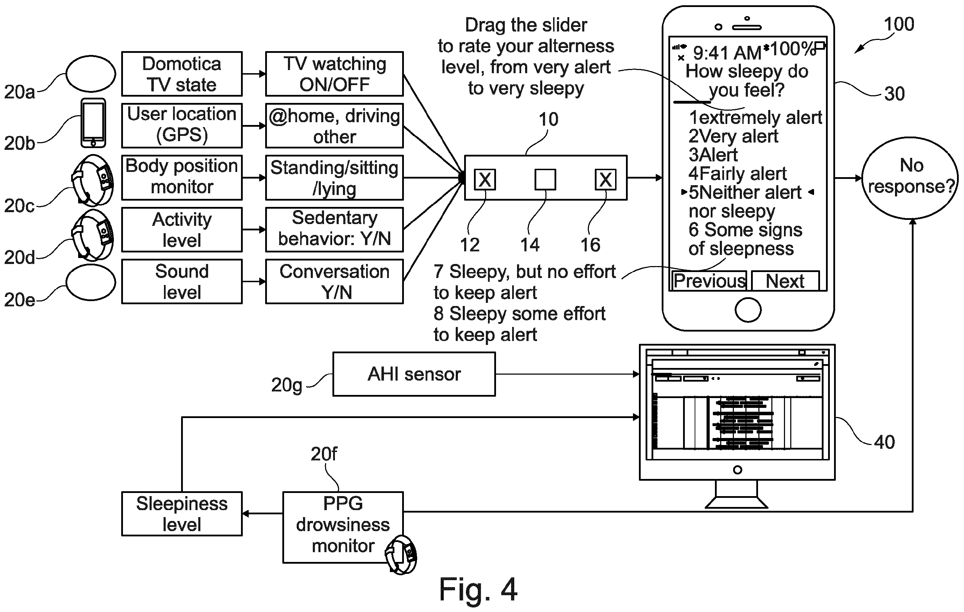

Fig. 4 illustrates a system 100 for sleepiness assessment. The system 100 comprises an apparatus 10 for sleepiness assessment, a sensor arrangement 20 comprising one or more sensors, a user interface 30, a workstation 40.

In the example of Fig. 4, the sensor arrangement 20 comprises a monitoring unit 20a, a location sensor 20b, a body position monitoring sensor 20c, a sedentary behavior detector 20d, a sound detector 20e, and a PPG drowsiness monitoring sensor 20f. The monitoring unit 20a may be wired or wireless connectable to a Smart Home interface (demotic) to get environmental information, such as TV on/off status, lights on/off settings, etc. The location sensor 20b may be used to detect whether the user is at home, driving a car, walking, etc. This can be achieved via GPS information of the smartphone shown in Fig. 4. The body position monitoring sensor 20c may be part of a smart watch and used to detect whether the user is standing, sitting, lying, etc. The sedentary behavior detector 20d may also be part of the smart watch and used to classify user behavior as 'sedentary' or 'non-sedentary/active'. The sound detector 20e may be used to detect whether the user is talking. The PPG drowsiness monitoring sensor 20f may be used to provide objective drowsiness measures. Although Fig. 4 may show a limited number of sensors by way of example, it can be appreciated that a greater or a fewer number of sensors may be employed for a given implementation.

Data collected from the sensor arrangement 20 is provided to the apparatus 10. From the collected data, the apparatus 10 may define the current situation (e.g. watching TV Yes or No) the user is engaged in. If the current situation matches one of a plurality of predefined situations used for situational sleepiness assessment, the apparatus 10 may send a notification to the user for a self-report of a user's current subjective sleepiness level via the user interface 30 shown as a smartphone app in Fig. 4. The user may select a score indicative of the subjective sleepiness level via the user interface 30. Collected input from the user upon a request for a subjective input ('How sleepy do you feel?') will be sent e.g. to a clinician dashboard displayed on the display of the workstation 40 and/or via feedback to the user in the smartphone app. If no input is provided by the user, objective drowsiness information from a wrist worn device (e.g. PPG drowsiness monitoring sensor 20f shown in Fig. 4) may be used as replacement.

The system shown in Fig. 4 may also be used for generating a treatment plan. For example, a sensor 20g, such as smart wearables, configured to measure the AHI of the user may be provided. A treatment plan generation device may be embodied in, or as, the workstation 40, which is configured to establish a treatment plan by linking nocturnal sleep behavior related to AHI to the impact of the sleep disturbance on daytime sleepiness in specific situations. e.g. using a trained machine learning model (e.g. convolutional neural networks). By using smart wearables capable of measuring the AHI and as such, a close relation between sleepiness at certain days or specific situations can be coupled to the AHI of the preceding night. Such information may be used to establish specific treatment plans, by linking nocturnal sleep behavior related to OSA (AHI), to the impact of the sleep disturbance on daytime sleepiness in specific situations, perhaps yielding alternative solutions or settings in the treatment plan (triaging engine). The specific manner to better triage could come over time, when sufficient data on sleepiness measures and treatment success can be evaluated. In this way, the sleepiness level in different identified situations can be correlated to nocturnal sleep measures. The clinical relevance of the sleepiness measure may significantly improve.

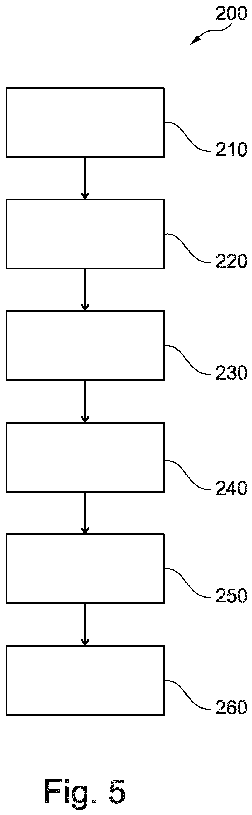

Fig. 5 illustrates a flow chart describing an exemplary method 200 for sleepiness assessment. The method 200 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the exemplary method may be implemented as an apparatus shown in Fig. 1.

At block 210, data indicative of an activity currently performed by a user is received. The received data may comprise one or more of the following exemplary data.

In some examples, the received data may comprise sensor data acquired by one or more on-body sensors including, but not limited to, inertial sensors (e.g. accelerometer, gyroscopes, pressure sensors, magnetic field sensors, or other inertial sensors), location sensors (e.g. GPS), physiological sensors (e.g. blood pressure cuff, electrocardiogram, spirometer, electrooculography, skin temperature sensor).

In some examples, the receive data may comprise sensor data acquired by one or more on-object sensors including, but not limited to, environmental sensors (e.g. thermometer, hygrometer, and/or energy sensor), location detectors (e.g. infrared), binary sensors, and tags (active RFID, RFID tags, NFC tags).

In some examples, the received data may comprise environmental information obtained via a SmartHome interface, such as TV on/off status, lights on/off settings, etc.

With the received data, it is possible to monitor a variety of activities including, but not limited to, aerobic exercises (e.g. walking, jogging, climbing, descending, running, or swimming), transportation (driving, cycling, or taking a bus), sedentary postures (e.g. sitting, lying, standing, or tilting), transitional activities (e.g. sit-to-stand, stand-to-walk, walk-to-run, or run-to-walk), daily lives (e.g. watching TV or cooking), and ball sports (e.g. playing football).

At block 220, based on the activity currently performed by the user, a current situation the user is engaging in is determined. For example, it may be determined that the user is watching TV, sitting inactive in a public place (e.g. a theatre or a meeting), running, or driving a car.

At block 230, it is determined whether the current situation matches one of a plurality of pre-determined situations used for situational sleepiness assessment. Examples of the pre-determined situations used for situational sleepiness assessment may include, but are not limited to, sitting and reading, watching TV, sitting inactive in a public place (e.g. a theatre or a meeting), as a passenger in a car for an hour without a break, lying down to rest in the afternoon when circumstances permit, sitting and talking to someone, sitting quietly after a lunch without alcohol, in a car while stopped for a few minutes in traffic.

At block 240, in response to the determination that the current situation matches one of the plurality of predetermined situations, a notification is sent to the user for a self-report of the user's subjective sleepiness level e.g. in form of a score. Alternatively or additionally, the user's objective sleepiness level is obtained from sensor data e.g. using a wearable (e.g. wrist worn) device with PPG. The objective drowsiness level from the sensor data may replace or enrich the user's self-reported subjective sleepiness level. For example, this information may further support the right moment for triggering notifications to the user to subjectively assess sleepiness level, or when the user does not respond to the request for information, replace the subjective input by the objective measure for that specific moment in time.

At block 250, based on the determined situation and the user's current subjective and/or objective sleepiness level, a situational sleepiness profile is generated which is indicative of a sleep disturbance on daytime sleepiness in specific situations. As discussed hereinbefore, on-body sensor(s), on-objection sensor(s) and/or monitoring unit(s) may be used to track the user's activity continuously. Therefore, various situations and associated subjective and/or objective sleepiness levels may be continuously collected and stored in the user's situational sleepiness profile. Therefore, the user's situational sleepiness profile may comprise previously and currently recorded situations and associated subjective and/or objective sleepiness levels.

At block 260, the generated situational sleepiness profile is provided, e.g. to a display device or to be stored in a database. The generated situational sleepiness profile is preferably useable to support sleep apnea diagnostics.

In some examples, if it is determined that the current situation has a duration exceeding a threshold, the method 200 may further comprise the step of sending a plurality of notifications to the user for a self-report of the user's subjective sleepiness levels throughout the duration of the current situation or obtaining the objective sleepiness levels throughout the duration of the current situation.

In some examples, the method 200 may further comprise the steps of determining, based on the received data, a moment that the user is less engaged in the current situation, and sending the notification to the user at the determined moment.

In some examples, the method 200 may comprise the steps of monitoring a delay between sending the notification and receiving the user's self-reported subjective sleepiness level, determining whether the delay exceeds a threshold, and adjusting, based on the delay, a moment for sending the notification to the user, in response to the determination that the delay exceeds the threshold.

In some examples, the method 200 may further comprise receiving data indicative of mental functioning of the user. The provided situational sleepiness profile may further comprise the information on mental functioning of the user. For example, information on mental function (e.g. stress level) can be estimated from measures with a connected wearable. This could be used as an indication of the 'burden' level that the patient is experiencing, replacing or enriching input from questions like 'How much is it a burden for you?', related to the suffering from the experienced sleep disorder.

In some examples, the method 200 may further comprise the following steps:
- receiving data indicative of activities the user performs from day to day over a predefined period;
- determining, based on the received data, a daily routine motion pattern of the user over the predefined period, wherein the daily routine motion pattern is indicative of an activity level over a day;
- determining, based on the daily routine motion pattern of the user, a low motion moment that happens repeatedly for a plurality of days over the predefined period, wherein at the low motion moment, the activity level is lower than a threshold;
- sending a notification to prompt the user for indicating whether the user did a nap at the low motion moment; and
- in response to a user's response indicating that the user did a nap at the low motion moment, providing information about the low motion moment to the situational sleepiness profile.

In some examples, the method 200 may further comprise the steps of receiving data comprising previous measurement of activities of the user and an associated situational sleepiness profile, and predicting a moment when sleepiness is likely to have changed from the previous measurement and send a notification to the user for a self-report of the user's sleepiness level at the determined moment.

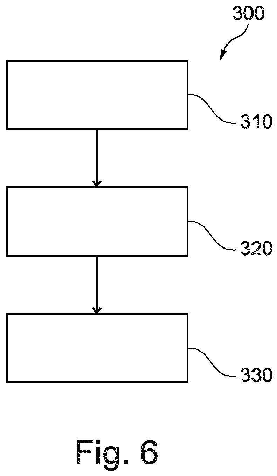

Fig. 6 illustrates a flow chart describing a method 300 for generating a treatment plan.

At block 310, an AHI of a user is measured e.g. using a wearable (e.g. wrist worn) device with PPG.

At block 320, a sleep disturbance on daytime sleepiness in specific situations is measured in a manner similar to the method described with respect to the example shown in Fig. 5.

At block 330, a treatment plan is established by linking nocturnal sleep behavior related to AHI to the impact of the sleep disturbance on daytime sleepiness in specific situations. A machine learning model (e.g. CNN) may be trained to generate the treatment plan based on the measured nocturnal sleep behavior and the sleep disturbance on daytime sleepiness in specific situations.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for sleepiness assessment, comprising:
- an input unit (12);
- a processing unit (14); and
- an output unit (16);
wherein the input unit is configured to receive data indicative of an activity currently performed by a user;
wherein the processing unit is configured to:
- determine, based on the activity currently performed by the user, a current situation the user is engaging in;
- determine whether the current situation matches one of a plurality of pre-determined situations used for situational sleepiness assessment;
- in response to the determination that the current situation matches one of the plurality of predetermined situations, send a notification to the user for a self-report of a user's current subjective sleepiness level and/or obtain a user's current objective sleepiness level from sensor data; and
- generate, based on the determined situation and the user's current subjective and/or current objective sleepiness level, a situational sleepiness profile indicative of a sleep disturbance on daytime sleepiness in specific situations; and
wherein the output unit is configured to provide the generated situational sleepiness profile.

2. The apparatus according to claim 1,
wherein if it is determined that the current situation has a duration exceeding a threshold, the processing unit is configured to send a plurality of notifications to the user for a self-report of the user's subjective sleepiness levels throughout the duration of the current situation and/or to obtain a plurality of objective sleepiness levels from the sensor data throughout the duration of the current situation.

3. The apparatus according to claim 1 or 2,
wherein the processing unit is configured to send the notification to the user for a self-report of a score indicative of the user's subjective sleepiness level.

4. The apparatus according to any one of the preceding claims,
wherein the processing unit is configured to obtain the user's objective sleepiness level from the sensor data, if no self-report of the user's subjective sleepiness level is received.

5. The apparatus according to any one of the preceding claims,
wherein the processing unit is configured to determine, based on the received data, a moment that the user is less engaged in the current situation; and
wherein the processing unit is configured to send the notification to the user at the determined moment.

6. The apparatus according to any one of the preceding claims,
wherein the processing unit is configured to:
- monitor a delay between sending the notification and receiving the user's self-reported subjective sleepiness level,
- determine whether the delay exceeds a threshold, and
- in response to the determination that the delay exceeds the threshold, adjust, based on the delay, a moment for sending the notification to the user.

7. The apparatus according to any one of the preceding claims,
wherein the input unit is configured to receive data indicative of mental functioning of the user; and
wherein the provided situational sleepiness profile further comprises the information on mental functioning of the user.

8. The apparatus according to any one of the preceding claims,
wherein the input unit is configured to receive data indicative of activities the user performs from day to day over a predefined period;
wherein the processing unit is configured to:
- determine, based on the received data, a daily routine motion pattern of the user over the predefined period, wherein the daily routine motion pattern is indicative of an activity level over a day;
- determine, based on the daily routine motion pattern of the user, a low motion moment that happens repeatedly for a plurality of days over the predefined period, wherein at the low motion moment, the activity level is lower than a threshold;
- send a notification to prompt the user for indicating whether the user did a nap at the low motion moment; and
- in response to a user's response indicating that the user did a nap at the low motion moment, provide information about the low motion moment to the situational sleepiness profile.

9. The apparatus according to any one of the preceding claims,
wherein the input unit is configured to receive data comprising previous measurement of activities of the user and an associated situational sleepiness profile; and
wherein the processing unit is configured to predict a moment when sleepiness is likely to have changed from the previous measurement and send a notification to the user for a self-report of the user's subjective sleepiness level at the determined moment.

10. The apparatus according to any one of the preceding claims,
wherein the data indicative of an activity currently performed by the user comprises:
- sensor data obtained from one or more sensors for monitoring an activity of the user;
- sensor data obtained from one or more sensors for monitoring an environmental parameter in a user's location;
- data obtained from one or more smart-home devices;
- data indicative of location information of the user; or
- any combination thereof.

11. A system (100) for generating a treatment plan, comprising:
- a sensor (20g) configured to measure an apnea hypopnea index, AHI, of a user;
- the apparatus according to any one of the preceding claims configured to measure a sleep disturbance on daytime sleepiness in specific situations; and
- a treatment plan generation device (40) configured to establish a treatment plan by linking nocturnal sleep behavior related to AHI to the impact of the sleep disturbance on daytime sleepiness in specific situations.

12. A method (200) for sleepiness assessment, comprising:
- receiving (210) data indicative of an activity currently performed by a user;
- determining (220), based on the activity currently performed by the user, a current situation the user is engaging in;
- determining (230) whether the current situation matches one of a plurality of pre-determined situations used for situational sleepiness assessment;
- in response to the determination that the current situation matches one of the plurality of predetermined situations, sending (240) a notification to the user for a self-report of the user's current sleepiness level and/or obtaining the user's current drowsiness level from sensor data; and
- generating (250), based on the determined situation and the user's current subjective and/or objective sleepiness level, a situational sleepiness profile indicative of a sleep disturbance on daytime sleepiness in specific situations; and
- providing (260) the generated situational sleepiness profile.

13. A method (300) for generating a treatment plan, comprising:
- measuring (310) an apnea hypopnea index, AHI, of a user;
- measuring (320) a sleep disturbance on daytime sleepiness in specific situations according to the method of claim 12; and
- establishing (330) a treatment plan by linking nocturnal sleep behavior related to AHI to the impact of the sleep disturbance on daytime sleepiness in specific situations.

14. A computer program product comprising instructions to cause the apparatus of any one of claims 1 to 10 to execute the steps of the method of claim 12, or comprising instructions to cause the system of claim 11 to execute the steps of the method of claim 13.

15. A computer-readable data carrier having stored there on the computer program product of claim 14.
